# EUROPEAN PATENT APPLICATION

(11) **EP 3 718 703 A1**
(43) Date of publication of application: **07.10.2020**
(21) Application number: 18884053.2
(22) Date of filing: 21.11.2018
(51) Int. Cl.: B25J 11/00, A61F 5/01, A61H 1/02, A61H 3/00

(54) **WEARABLE IMPLEMENT AND MOTION ASSISTANCE DEVICE**

(30) Priority: 28.11.2017 JP 2017227997
(71) Applicant: Panasonic Corporation, Kadoma-shi, Osaka 571-8501 (JP)
(72) Inventor: MORIKAWA, Fumitaka, Osaka-shi, Osaka 540-6207 (JP); HABASAKI, Shohei, Osaka-shi, Osaka 540-6207 (JP)
(74) Representative: Vigand, Philippe
(86) International application number: PCT/JP2018/042980
(87) International publication number: WO 2019/107244

(57) **Abstract**

A movement assistance device (1) that is a wearable implement includes: a frame (12) that is to be fitted onto a wearer and extends from a lower back to a right side of hips and a left side of the hips of the wearer when fitted onto the wearer. The frame (12) includes a pair of fitting portions (13) that extend to a vicinity of the right side of the hips and the left side of the hips of the wearer, and a space between the pair of fitting portions (13) widens from the lower back toward an abdomen of the wearer. For example, each of the pair of fitting portions (13) may be an end of the frame (12). Moreover, each of the pair of fitting portions (13) has an approximately planar surface that faces the right side of the hips or the left side of the hips of the wearer.

## Description

### [Technical Field]

The present invention relates to a wearable implement and a movement assistance device.

### [Background Art]

There are movement assistance devices that are fitted onto persons and assist movements of the persons fitted with the movement assistance device (hereinafter, also referred to as wearers). For example, when a wearer moves to lift a heavy object, a movement assistance device generates part of the force required for the movement to reduce the force to be generated by the wearer. In this way, the movement assistance device assists the movement of the wearer by covering part of the force to be generated by the wearer.

The movement assistance devices are roughly divided into two types: one is a grounded type, and the other is a non-grounded type. A grounded-type movement assistance device is used in a state that the movement assistance device is in contact with the ground surface. A wearer does not need to support the weight of the grounded-type movement assistance device, because the wearer rides on the movement assistance device which is in contact with the ground surface.

In contrast, a non-grounded type movement assistance device is fitted onto part of the body of the wearer and used without being in contact with the ground surface. The wearer needs to support the weight of the non-grounded type movement assistance device, because such a movement assistance device is fitted onto the body of the wearer. Moreover, the non-grounded-type movement assistance device is fitted onto the wearer by, for example, being slung over the shoulders of the wearer or being wound around the hips of the wearer, and therefore is more likely to limit free movements of the wearer compared with the case of the grounded-type movement assistance device.

One example of the movement assistance devices is a movement assistance device that assists movements of the hip joints of the wearer. The movement assistance device for assisting movements of the hip joints of the wearer includes separated frames: a frame to be fitted onto a thigh of the wearer, and a frame to be fitted onto the upper body of the wearer. Moreover, an actuator is provided in a part which connects the frames with each other, and one frame is moved relative to the other frame.

The movement assistance device for assisting movements of the hip joints of the wearer needs to ensure that the wearer can easily open and close the right and left hip joints. This is because the movement assistance device needs to be used in a state that a hip joint or hip joints are opened.

Patent Literature (PTL) 1 discloses a non-grounded-type movement assistance device. This movement assistance device assists a forward bending action of a wearer.

PTL 2 discloses a movement assistance device that includes hinges in part of a back frame to ensure that hip joints can be opened.

Non Patent Literature (NPL) 1 discloses a movement assistance device that includes hinges in part of the frames to be fitted onto thighs of a wearer to ensure that the hip joints can be easily opened.

### [Citation List]

### [Patent Literature]

[PTL 1] Japanese Patent No. 5526444
[PTL 2] International publication No. 2016/021103

### [Non Patent Literature]

[NPL 1] CYBERDYNE, INC., HAL LUMBER TYPE FOR LABOR SUPPORT, [online], [searched on November 27, 2017], on the Internet, <URL:https://www.cyberdyne.jp/products/Lumbar_LaborSupport.h tml>

### [Summary of Invention]

### [Technical Problem]

The movement assistance device disclosed in PTL 1 includes frames etc. made of a rigid member in order to maintain the structure of the movement assistance device. Especially, the plates disposed in the vicinity of the sides of the hips of the wearer may limit free motion of the legs of the wearer.

Furthermore, the movement assistance device disclosed in PTL 2 includes hinges to ensure that hip joints can be opened, and thus the weight and the component count of the movement assistance device increase. If the weight of the movement assistance device increases, the wearer needs large force to support the weight of the movement assistance device. An increase in component count of the movement assistance device will also greatly increase the cost of the movement assistance device, for example.

Moreover, the movement assistance device disclosed in NPL 1 may interfere with the body of the wearer in the vicinity of the hip joints of the wearer.

Accordingly, the movement assistance device disclosed in each of the above documents needs to prevent interference between the wearer and the wearable implement in the vicinity of the hip joints while reducing the weight and the component count.

The present invention is conceived in order to solve the aforementioned existing problems and has an object to provide a wearable implement etc. that prevents interference between a wearer and the wearable implement in the vicinity of the hip joints while reducing the weight and the component count.

### [Solution to Problem]

In order to solve the aforementioned problems, a wearable implement according to one aspect of the present invention includes: a rigid member that is to be fitted onto a wearer and extends from a lower back to a right side of hips and a left side of the hips of the wearer when fitted onto the wearer. The rigid member includes a pair of fitting portions that extend to a vicinity of the right side of the hips and the left side of the hips of the wearer, and a space between the pair of fitting portions widens from the lower back toward an abdomen of the wearer.

### [Advantageous Effects of Invention]

The movement assistance device according to one aspect of the present invention can prevent interference between the wearer and the wearable implement in the vicinity of the hip joints while reducing the weight and the component count. Furthermore, protrusion of the wearable implement in a front direction of the wearer can be prevented.

### [Brief Description of Drawings]

[FIG. 1]

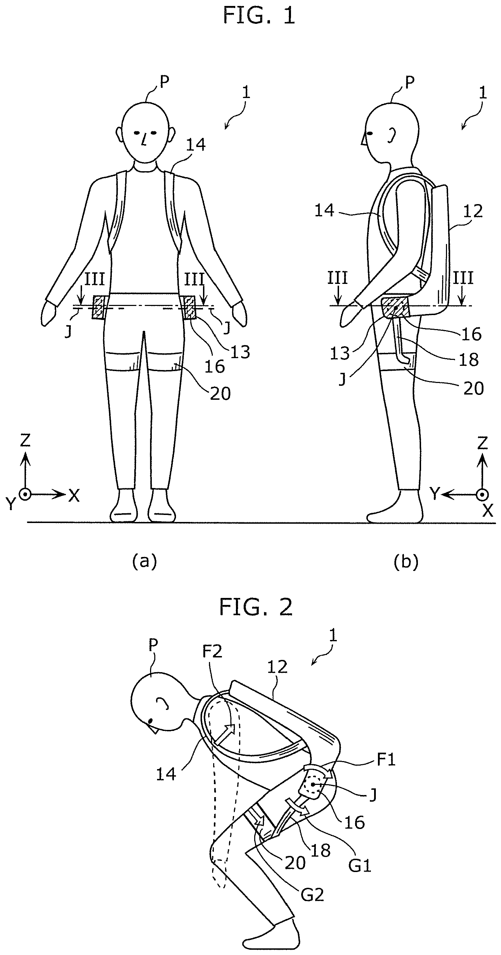

   FIG. 1 is front and side views illustrating external views of a movement assistance device according to an embodiment.
[FIG. 2]
   FIG. 2 is an explanatory diagram illustrating a method of assisting a movement by the movement assistance device according to the embodiment.
[FIG. 3]

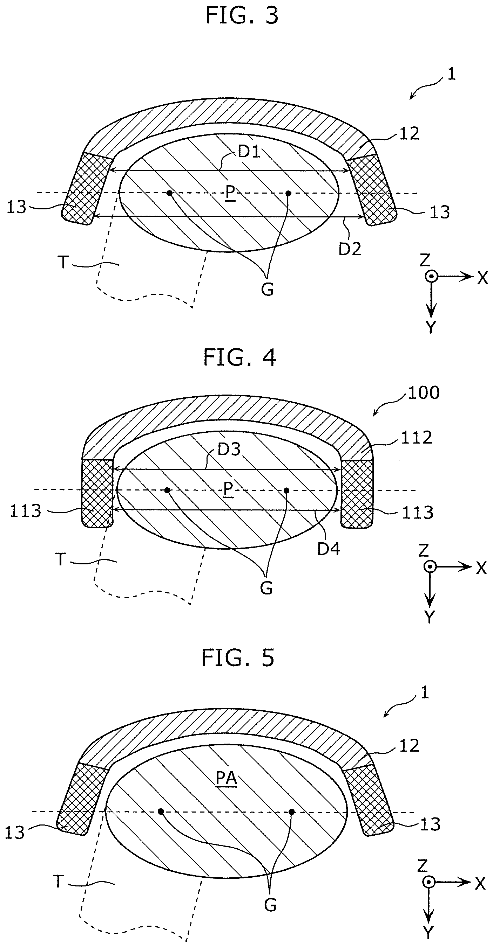

   FIG. 3 is a first explanatory diagram schematically illustrating a cross-sectional shape of a frame according to the embodiment.
[FIG. 4]
   FIG. 4 is an explanatory diagram schematically illustrating a cross-sectional shape of a frame according to a related art.
[FIG. 5]
   FIG. 5 is a second explanatory diagram schematically illustrating a cross-sectional shape of the frame according to the embodiment.
[FIG. 6]

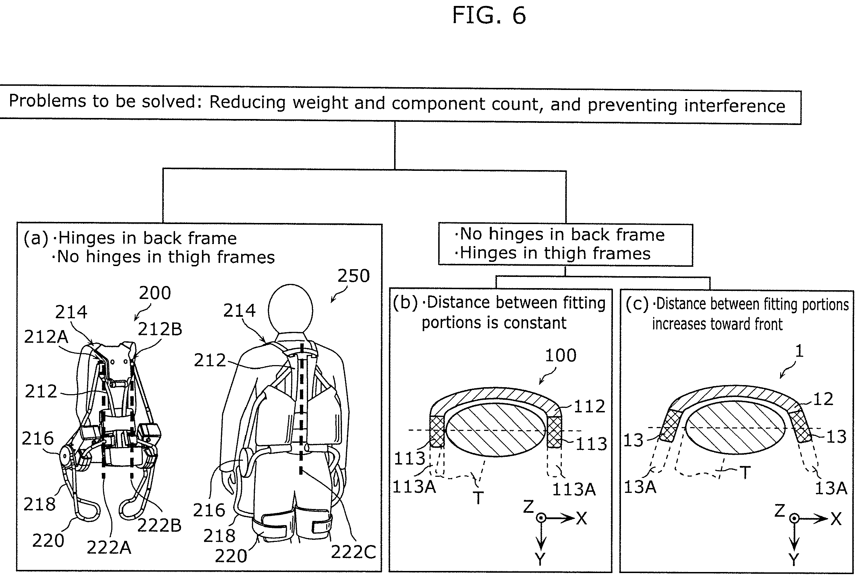

   FIG. 6 is an explanatory diagram illustrating comparisons of the movement assistance device according to the embodiment and the movement assistance device according to the related art.

### [Description of Embodiments]

A wearable implement according to one aspect of the present invention includes: a rigid member that is to be fitted onto a wearer and extends from a lower back to a right side of hips and a left side of the hips of the wearer when fitted onto the wearer. The rigid member includes a pair of fitting portions that extend to a vicinity of the right side of the hips and the left side of the hips of the wearer, and a space between the pair of fitting portions widens from the lower back toward an abdomen of the wearer.

According to the above aspect, the space between fitting portions of the wearable implement increases from the lower back to the abdomen of the wearer. Thus, the movement assistance device can avoid disturbing movements of the wearer, and can allow a change in shape or size of the body of the wearer to some extent. Accordingly, the wearable implement can prevent interference between the wearer and the wearable implement in the vicinity of the hip joints while reducing the weight and the component counts. Furthermore, protrusion of the wearable implement in the front direction of the wearer can be prevented.

For example, each of the pair of fitting portions may be an end of the rigid member.

According to this aspect, the fitting portions of the wearable implement, which are the ends of the rigid member, avoid disturbing movements of the wearer. Moreover, the rigid member is fitted onto the wearer and extends from the lower back to the right side of the hips and left side of the hips of the wearer when fitted onto the wearer. In other words, the rigid member does not reach the abdomen or extend ahead of the sides of the hips of the wearer. If the rigid member reaches ahead of the sides of the hips of the wearer, interference between the wearer and the rigid member may occur. The wearable implement according to one aspect of the present invention can avoid, beforehand, disturbance of movements of the wearer resulting from such interference.

For example, each of the pair of fitting portions may have an approximately planar surface that faces the right side of the hips or the left side of the hips of the wearer.

According to the above aspect, the wearable implement prevents a side of the hips of wearer or clothes on a side of the hips of wearer from being caught on the fitting portion and prevents disturbing movements of the wearer. The wearable implement also prevents an injury or causing a pain to the wearer which may be caused when a side of the hips of the wearer is pushed by the fitting portion. If the wearable implement has uneven surfaces facing the sides of the hips, a side of the hips of the wearer or clothes on a side of the hips of the wearer may be caught on the fitting portion, and thus movements of the wearer may be disturbed. Moreover, a side of the hips of the wearer may be pushed by the fitting portion, and this may cause an injury or a pain to the wearer. Therefore, the wearable implement can be fitted onto the wearer while maintaining the degree of freedom of movements of the wearer and avoiding an injury etc. to the wearer.

Moreover, the movement assistance device according to one aspect of the present invention includes: the wearable implement; a belt connected to the wearable implement to fit the wearable implement onto an upper body of the wearer; a pair of thigh fitting portions to be fitted onto a right thigh and a left thigh of the wearer; a connecting portion that connects one of the pair of fitting portions on a right side or a left side to a corresponding one of the pair of thigh fitting portions on the right side or the left side; and a motor that generates force in a direction that causes the rigid member to rotate relative to the connecting portion about one of the pair of fitting portions serving as a rotation axis to apply, on the upper body of the wearer via the belt, force in a direction from the abdomen to a back of the wearer.

According to this aspect, the movement assistance device can be fitted onto the wearer while maintaining the degree of freedom of the movements, as in the wearable implement.

Hereinafter, an embodiment is specifically described with reference to the drawings.

Note that the following embodiment describes a general or specific example of the present invention. The numerical values, shapes, materials, structural elements, the arrangement and connection of the structural elements, steps, the processing order of the steps etc. shown in the following embodiment are mere examples, and are not intended to limit the scope of the present invention. Furthermore, among the structural elements in the following embodiment, structural elements not recited in the independent claim indicating the broadest concept of the present invention are described as optional structural elements.

### [Embodiment]

In the present embodiment, movement assistance device 1 that is a wearable implement that prevents reduction in degree of freedom of movements of a wearer will be described.

FIG. 1 is front (a) and side (b) views illustrating external views of movement assistance device 1 according to the present embodiment. FIG. 1 illustrates movement assistance device 1 and wearer P who is fitted with movement assistance device 1. However, movement assistance device 1 does not include wearer P. Note that the following description includes description using the coordinate axes indicated in the drawings. In such description, there are cases where the positive direction of the Z axis is referred to as up, and the negative direction of the Z axis is referred to as down. Furthermore, there are cases where the positive direction of the Y axis is referred to as front, and the negative direction of the Y axis is referred to as rear. Moreover, the word "right" or "left" basically means the right or left seen from wearer P.

As illustrated in FIG. 1, movement assistance device 1 is a non-grounded-type movement assistance device that is fitted onto wearer P and extends over the back, the hips (hip area), and the thighs of wearer P when fitted onto the wearer. Movement assistance device 1 is an example of the wearable implement.

Movement assistance device 1 includes frame 12, belt 14, motor 16, connecting portion 18, and thigh fitting portion 20.

Frame 12 is a rigid member, i.e., a frame having rigidity, to be fitted onto wearer P and extends from the lower back to the right and left sides of the hips when fitted onto the wearer. Frame 12 does not change its shape, except for slight bending. In other words, part of frame 12 extending from the lower back to the right and left sides of the hips of wearer P does not include a movable mechanism, such as a hinge mechanism. The size of frame 12 is approximately 40 cm to 60 cm in the right-left direction, and approximately 40 cm to 60 cm in the up-down direction, for example. Frame 12 is made of a lightweight metal, such as aluminum, or a synthetic material, such as a cured resin and carbon fibers.

Inside frame 12, a battery for driving motor 16, a peripheral circuit, wirings, etc. are included.

Frame 12 includes fitting portion 13 which is part of frame 12. Fitting portions 13 are part of frame 12 that extend to a vicinity of the right and left sides of the hips of wearer P when fitted onto the wearer. Each of fitting portions 13 is rotatably connected to connecting portion 18. Fitting portion 13 functions as a joint that connects frame 12 and connecting portion 18. Fitting portion 13 may include an angle sensor (for example, an encoder including a Hall effect sensor) which detects a rotation angle of frame 12 with respect to connecting portion 18.

Note that fitting portion 13 may be an end of frame 12. When fitting portion 13 is an end of frame 12, frame 12 does not reach the abdomen or extend ahead of the sides of the hips of the wearer. If frame 12 extends ahead of the sides of the hips from wearer P, the portions of frame 12 which position ahead of the sides of the hips may disturb the movement of the wearer. However, this can be avoided beforehand.

Moreover, surfaces of fitting portions 13 that face right and left sides of the hips of wearer P may be approximately planar. This prevents a side of the hips of wearer P or clothes on a side of the hips of wearer P from being caught on fitting portion 13 and prevents disturbing movements of wearer P. This also prevents an injury or causing a pain to the wearer which may be caused when a side of the hips of wearer is pushed by fitting portion 13.

Belt 14 is a belt portion connected to frame 12 and is used to fit frame 12 onto wearer P. One end of belt 14 is connected to the upper end portion of frame 12. Moreover, the other end of belt 14 is connected to frame 12 in the vicinity of the lower back of wearer P. Belt 14 includes belt 14 on the left side and belt 14 on the right side. Belt 14 on the left side is connected from an upper end of frame 12 to another portion of frame 12 and passes through left shoulder, left chest, and under the left arm of wearer P. Belt 14 on the right side is connected from an upper end of frame 12 to another portion of frame 12 and passes through right shoulder, right chest, and under the right arm of wearer P. The length of belt 14 is adjustable in accordance with the shape of the body of wearer P, and belt 14 fits closely onto the body of wearer P with appropriate tightening power. The size (length) of belt 14 is approximately from 50 cm to 60 cm, for example.

Motor 16 generates force in a direction that causes frame 12 to rotate relative to connecting portion 18 about fitting portion 13 of frame 12, and fitting portion 13 serves as a rotation axis. More specifically, motor 16 generates force in a direction that causes frame 12 to rotate relative to connecting portion 18 about rotation axis J that passes through in the through-thickness direction of fitting portion 13. This force produces force on the upper body of wearer P via frame 12 and belt 14 in a direction from the abdomen toward the back of wearer P. The force generated by motor 16 may be controlled by an output value of the angle sensor that detects the rotation angle of frame 12 relative to connecting portion 18. Movement assistance device 1 has the information in advance that shows correspondence between the output values of the angle sensor and force to be generated by motor 16 (specifically, a relational expression, a correspondence table, and so on). Movement assistance device 1 determines and controls the force to be generated by motor 16 based on the information.

Connecting portion 18 is a rigid member, i.e., a frame having rigidity, and connects fitting portion 13 and thigh fitting portion 20. Connecting portion 18 includes connecting portion 18 on the left side and connecting portion 18 on the right side. Connecting portion 18 on the left side connects fitting portion 13 on the left side and thigh fitting portion 20 on the left side. Connecting portion 18 on the right side connects fitting portion 13 on the right side and thigh fitting portion 20 on the right side. One end of each connecting portion 18 is connected to fitting portion 13 and extends downward from the one end along a leg of wearer P, and the other end is connected to thigh fitting portion 20. The size of connecting portion 18 is approximately 20 cm to 30 cm, for example. Connecting portion 18 is made of a lightweight metal, such as aluminum, or a synthetic material, such as a cured resin or carbon fibers.

Thigh fitting portion 20 is to be fitted onto a thigh of wearer P. One thigh fitting portion 20 is fitted onto the right thigh, and another thigh fitting portion 20 is fitted onto the left thigh of wearer P. Each thigh fitting portion 20 is fitted onto wearer P by being wounded around the thigh, for example. Note that each thigh fitting portion 20 may be a rod or a plate that abuts the thigh of wearer P.

FIG. 2 is an explanatory diagram illustrating a method of assisting a movement by movement assistance device 1 according to the present embodiment.

FIG. 2 illustrates that wearer P who is fitted with movement assistance device 1 is in a half-sitting posture that is an intermediate posture for changing from a squatting posture to a standing posture (the posture illustrated in FIG. 1). For example, such a movement is made when wearer P tries to lift an object (not illustrated) placed on a floor.

FIG. 2 also shows force F1 applied to frame 12 by motor 16 and force G1 applied to connecting portion 18 by motor 16. Force F1 and force G1 are determined based on the information that shows correspondence between the output values of the angle sensor that are held by movement assistance device 1 and the force to be generated by motor 16.

Force F1 becomes force F2 that pushes the chest of wearer P in a direction from the abdomen toward the back of wearer P via frame 12 and belt 14, and acts on the body of wearer P. Moreover, force G1 becomes force G2 that pushes the thigh of wearer P downward or rearward via connecting portion 18 and thigh fitting portion 20, and acts on the body of wearer P.

In this way, movement assistance device 1 assists the standing-up movement of wearer P by controlling the force to be generated by motor 16 based on an output value of the angle sensor when wearer P tries to stand up.

Hereinafter, the shape of frame 12 will be described in more detail.

FIG. 3 is a first explanatory diagram schematically illustrating a cross-sectional shape of frame 12 according to the present embodiment. The cross-sectional shapes illustrated in FIG. 3 are a cross section of frame 12 and a cross section of the hips of wearer P taken along line III-III in FIG. 1.

As illustrated in FIG. 3, the space between a pair of fitting portions 13 on the right and left sides widens from the lower back toward the abdomen of wearer P. In other words, the space between the pair of fitting portions 13 on the right and left sides widens toward the front of wearer P. That is, the distance between the pair of fitting portions 13 on the right and left sides increases toward the positive direction of the Y axis. More specifically, distance D2 between positions of the pair of fitting portions 13 relatively closer to the abdomen is greater than distance D1 between positions of the pair of fitting portions 13 closer to the back of wearer P.

With this structure, movement assistance device 1 has advantages that disturbance of movements, more specifically, motion of the legs, of wearer P can be avoided, and change in shape or size of the body of wearer P can be allowed to some extent. These advantages will be described in the following.

### (1) Regarding motion of legs

The advantage that movement assistance device 1 avoids disturbing motion of the legs of wearer P will be described with reference to FIG. 3 and FIG. 4. FIG. 4 is an explanatory diagram schematically illustrating a cross-sectional shape of frame 112 according to a related art, which is used for comparison. FIG. 4 illustrates a cross section of frame 112 according to the related art, and a cross section of the hips of wearer P. Frame 112 is, for example, a frame of a conventional movement assistance device corresponding to frame 12 of movement assistance device 1 according to the present embodiment.

As illustrated in FIG. 4, the space between a pair of fitting portions 113 positioned on the right and left sides and included in frame 112 remains the same regardless of whether the space is between positions closer to the lower back of wearer P or closer to the abdomen of wearer P. In other words, the distance between positions of the pair of fitting portions 113 on the right and left sides in the direction of the Y axis remains the same. More specifically, distance D4 between positions of the pair of fitting portions 113 relatively closer to the abdomen is the same as distance D3 between positions of the pair of fitting portions 113 relatively closer to the back of wearer P.

In this case, for example, when thigh T on the right side of wearer P moves in a right front direction seen from wearer P (the positive direction of the Y axis and the negative direction of the X axis) with respect to trochanter major G, that is, when wearer P flexes at the hip joint and turns the right leg outward, thigh T and fitting portion 113 may interfere with each other and the movement of thigh T may be disturbed, or an injury or a pain in thigh T may be caused by being pushed by fitting portion 113.

In contrast, the space between the pair of fitting portions 13 of movement assistance device 1 according to the present embodiment widens from the lower back toward the abdomen as illustrated in FIG. 3. Thus, fitting portion 13 does not interfere with thigh T even when thigh T moves as described above. Therefore, this avoids disturbing movements of thigh T and an injury or a pain in thigh T resulting from being pushed by fitting portion 13.

### (2) Regarding shape or size of body of wearer P

The advantage that movement assistance device 1 allows change in shape or size of the body of wearer P to some extent will be described with reference to FIG. 3 and FIG. 5. FIG. 5 is a second explanatory diagram schematically illustrating a cross-sectional shape of frame 12 according to the present embodiment. FIG. 5 illustrates a cross section of frame 12 and a cross section of the hips of wearer PA when wearer PA is fitted with movement assistance device 1. Here, wearer PA has a larger body than wearer P illustrated in FIG. 3.

As illustrated in FIG. 5, the space between the pair of fitting portions 13 of movement assistance device 1 widens from the lower back toward the abdomen. Thus, interference between a side of the hips of wearer PA and fitting portion 13 can be avoided even when movement assistance device 1 is fitted onto wearer PA. Moreover, even when thigh T moves as described above, interfering with fitting portion 13 can be avoided. Therefore, this avoids disturbing movements of thigh T, and an injury to or a pain in thigh T resulting from being pushed by fitting portion 13.

Next, movement assistance device 1 according to the present embodiment will be described by comparing movement assistance device 1 with the movement assistance devices according to related arts.

FIG. 6 is an explanatory diagram illustrating comparisons of movement assistance device 1 according to the present embodiment and movement assistance devices 200, 250, and 100 according to related arts. FIG. 6 also illustrates features of each movement assistance device that are relevant to the problems to be solved in the present invention. Here, movement assistance devices 200 and 250 according to a related art are examples of the movement assistance device disclosed in PTL 2, and movement assistance device 100 according to a related art is an example of the movement assistance device disclosed in NPL 1.

Movement assistance device 200 illustrated in (a) in FIG. 6 is a movement assistance device that includes hinges in part of a back frame to ensure that hip joints can be opened. Movement assistance device 200 includes frame (i.e., back frame) 212, belt 214, motor 216, connecting portion 218, and thigh fitting portion 220. These structural elements included in movement assistance device 200 have the same functions as the functions of the structural elements having the same names in movement assistance device 1.

However, frame 212 differs from frame 12 of movement assistance device 1 in that frame 212 includes hinges 212A and 212B. Frame 212 can follow a movement of the body of the wearer because the portion to be fitted onto the left side of the body and the portion to be fitted onto the right side of the body of the wearer respectively rotate about axis 222A and 222B using hinges 212A and 212B.

Movement assistance device 200 can avoid a pain, etc. in a thigh of the wearer that may arise when the thigh of the wearer is pushed by movement assistance device 200.

On the other hand, the component count of movement assistance device 200 increases by including hinges 212A and 212B, for example. Moreover, thigh fitting portion 220 protrudes in the front direction seen from the wearer.

Similar to movement assistance device 200, movement assistance device 250 illustrated in (a) in FIG. 6 includes, frame 212, belt 214, motor 216, connecting portion 218, and thigh fitting portion 220. Movement assistance device 250 is illustrated in a state where the movement assistance device is fitted onto a wearer. Although movement assistance device 250 differs from movement assistance device 200 in that the rotation axis of frame 212 is one axis, which is axis 222C, other features are the same as the features of movement assistance device 200.

Movement assistance device 100 illustrated in (b) in FIG. 6 is movement assistance device 100 illustrated in FIG. 4. In movement assistance device 100, the space between a pair of fitting portions 113 positioned on the right and left sides and included in frame 112 is the same regardless of whether the space is between positions closer to the lower back or closer to the abdomen of the wearer.

As described above, movement assistance device 100 may cause interference between a thigh of the wearer and fitting portion 113, thereby causing disturbance of a movement of the thigh, or an injury or a pain in the thigh when the thigh is pushed by fitting portion 113.

Moreover, movement assistance device 100 may have a structure including fitting portions 113A by extending fitting portions 113 further forward. In this case, however, fitting portions 113A protrude in the front direction seen from the wearer, and the areas that interfere with the thighs increases.

Movement assistance device 1 illustrated in (c) in FIG. 6 is movement assistance device 1 according to the present embodiment. In movement assistance device 1, the space between a pair of fitting portions 13 on the right and left sides included in frame 12 widens from the lower back toward the abdomen of the wearer.

Movement assistance device 1 can avoid a pain, etc. in a thigh of the wearer that may arise when the thigh of the wearer is pushed by movement assistance device 1. Moreover, movement assistance device 1 may have a structure including fitting portions 13A by extending fitting portions 13 further forward. Such a structure also has a feature of preventing protrusion of fitting portions 13A in the front direction seen from the wearer and interference with the thighs.

As described above, in the wearable implement according to the present embodiment, the space between a pair of the fitting portions increases from the lower back to the abdomen of the wearer. Thus, the movement assistance device can avoid disturbing movements of the wearer, and can allow a change in shape or size of the body of the wearer to some extent. Accordingly, the wearable implement can prevent interference between the wearer and the wearable implement in a vicinity of the hip joints while reducing the weight and the component counts. Furthermore, protrusion of the wearable implement in the front direction of the wearer can be prevented.

Moreover, the fitting portions of the wearable implement, which are the ends of the rigid member, avoid disturbing movements of the wearer. Moreover, the rigid member is fitted onto the wearer and extends from the lower back to the right and left sides of the hips of the wearer when fitted onto the wearer. In other words, the rigid member does not reach the abdomen or extend ahead of the sides of the hips of the wearer. If the rigid member reaches ahead of the sides of the hips of the wearer, interference between the wearer and the rigid member may occur. The wearable implement according to one aspect of the present invention can avoid, beforehand, disturbance of movements of the wearer resulting from such interference.

The wearable implement also prevents a side of the hips of the wearer or clothes on a side of the hips of the wearer from being caught on the fitting portion and prevents disturbing movements of the wearer. The wearable implement also prevents an injury or causing a pain to the wearer which may be caused when a side of the hips of wearer is pushed by the fitting portion. If the wearable implement has uneven surfaces facing the sides of the hips, a side of the hips of the wearer or clothes on a side of the hips of the wearer may be caught on the fitting portion and the wearable implement may disturb the movements of the wearer, or a side of the hips of the wearer may be pushed by the fitting portion and the wearable implement may injure or cause a pain to the wearer. Therefore, the wearable implement can be fitted onto the wearer while maintaining the degree of freedom of movements of the wearer and avoiding an injury, etc. to the wearer.

Although the wearable implement, etc. according to one or more aspects of the present invention have been described above based on the embodiment, the present invention is not limited to the above embodiment. Various modifications of the present embodiment as well as embodiments resulting from combinations of structural elements of the different embodiments that may be conceived by those skilled in the art may be included within the scope of the one or more aspects as long as these do not depart from the essence of the present invention.

### [Industrial Applicability]

The present invention is applicable to a wearable implement that prevents reduction in the degree of freedom of movements of a wearer. More specifically, the present invention is available to a power assist suit, a movement assistance device, a walk assist device, and so on.

### [Reference Signs List]

- 1, 100, 200, 250: movement assistance device
- 12, 112, 212: frame
- 13, 13A, 113, 113A: fitting portion
- 14, 214: belt
- 16, 216: motor
- 18, 218: connecting portion
- 20, 220: thigh fitting portion
- 212A, 212B: hinge
- 222A, 222B, 222C: axis
- F1, F2, G1, G2: force
- G: trochanter major
- J: rotation axis
- P, PA: wearer
- T: thigh

## Claims

1. A wearable implement, comprising:
a rigid member that is to be fitted onto a wearer and extends from a lower back to a right side of hips and a left side of the hips of the wearer when fitted onto the wearer, wherein
the rigid member includes a pair of fitting portions that extend to a vicinity of the right side of the hips and the left side of the hips of the wearer, and a space between the pair of fitting portions widens from the lower back toward an abdomen of the wearer.

2. The wearable implement according to claim 1, wherein
each of the pair of fitting portions is an end of the rigid member.

3. The wearable implement according to claim 1 or 2, wherein
each of the pair of fitting portions has an approximately planar surface that faces the right side of the hips or the left side of the hips of the wearer.

4. A movement assistance device, comprising:
the wearable implement according to any one of claims 1 to 3;
a belt connected to the wearable implement to fit the wearable implement onto an upper body of the wearer;
a pair of thigh fitting portions to be fitted onto a right thigh and a left thigh of the wearer;
a connecting portion that connects one of the pair of fitting portions on a right side or a left side to a corresponding one of the pair of thigh fitting portions on the right side or the left side; and
a motor that generates force in a direction that causes the rigid member to rotate relative to the connecting portion about one of the pair of fitting portions serving as a rotation axis to apply, on the upper body of the wearer via the belt, force in a direction from the abdomen to a back of the wearer.
